# EUROPEAN PATENT SPECIFICATION

(11) **EP 0 516 851 B1**
(45) Date of publication and mention of the grant of the patent: **19.06.1996**
(21) Application number: 91905340.5
(22) Date of filing: 27.02.1991
(51) Int. Cl.: C07H 19/00, C07H 15/18, C07K 14/70, C07K 5/04, C07C 229/00, C07D 207/00, A61K 31/70, A61K 31/40, A61K 31/195

(54) **PHYSIOLOGICALLY ACTIVE SUBSTANCE WELL CAPABLE OF PERMEATING BIOMEMBRANE**
PHYSIOLOGISCH AKTIVE SUBSTANZEN, DIE GUT ZUM DURCHDRINGEN BIOLOGISCHER MEMBRANEN BEFÄHIGT SIND
SUBSTANCE PHYSIOLOGIQUEMENT ACTIVE CAPABLE DE PASSER A TRAVERS UNE BIOMEMBRANE

(30) Priority: 28.02.1990 JP 50116/90
(43) Date of publication of application: 09.12.1992
(60) Divisional application: 95105215.8
(73) Proprietor: TEIKOKU SEIYAKU KABUSHIKI KAISHA, Okawa-gun Kagawa-ken (JP)
(72) Inventor: KITAGAWA, Kouki, Itano-gun, Tokushima 771-02 (JP); HIBI, Toru, Kawasaki-shi, Kanagawa 213 (JP); Mizobuchi, Noriko, Okawa-gun, Kagawa 769-25 (JP)
(74) Representative: Tiedtke, Harro, Dipl.-Ing.
(86) International application number: JP9100256
(87) International publication number: WO9113081

(56) References cited:
- EP-A- 187 052
- EP-A- 257 378
- DE-A- 2 352 618
- JP-A-51 043 769
- JP-A-61 109 766
- JP-A-63 051 384
- JP-A-63 295 590
- US-A- 4 273 704
- No further relevant documents have been disclosed.
- Il Farmac 0-Ed Sc 34(6) 496-506, 1979

## Description

The present invention concerns a physiologically active substance having high biomembrane permeability, more specifically, blood-brain barrier (BBB) permeability. Since the physiologically active substance according to the present invention has a high blood-brain barrier permeability, it can provide a sufficient intraventricular physiological activity even by means of subcutaneous administration or intravenous injection which is simpler than intraventricular administration.

Morphine as an opioid alkaloid in a poppy has been known from long since to have an effective analgesic effect and has been utilized with an aim of mitigating a keen pain typified in the terminal stage of cancer. However, since morphine has side-effects such as addiction, dependence, depression of respiration and constipation, it has drawbacks that its use is rather restricted and the way of use is difficult. In view of the above, it has been strongly demanded for the development of an analgesic having an analgesic effect equal or superior to morphine and showing no addiction such as morphine.

On the other hand, enkephalin which is an endogenic morphine-like substance (endogenic opioid) was found by J.Hughes et. al, in 1975 and it has been expected as a substance capable of substituting for morphine. However, it has been found that enkephalin and derivatives thereof involve drawbacks in that
(1) they are peptides and, accordingly, decomposed rapidly by enzymes in a living body,
(2) they are poor in the BBB permeability and have to be administrated directly into a brain, and
(3) they show similar side-effects to those of morphine such as addiction or dependence although they are endogenic substance.

For overcoming the foregoing drawbacks of the enkephalins, various studies have been made also at present but most of them have been directed to the enhancement of analgesic activity and moderation of the side-effects of the enkephalins in the vitro test or intraventricular administration test, as well as development for derivatives having high resistivity to decomposition by in vivo enzymes. The US-A-4 273 704 discloses N-adamantane substituted tetrapeptide amides, wherein the tetrapeptides are analoguous to enkephalin. In view of the foregoing situations, the present inventors have tried an improvement for the BBB permeability, which has not yet been studied by so much. Further, it has been expected that if the BBB permeability of the enkephalins can be improved, the BBB permeability of other substances with poor BBB permeability can also be increased to extend the application region.

The physiologically active substance well capable of permeating biomembrane according to the present invention has a feature in that it is represented by the general formula: 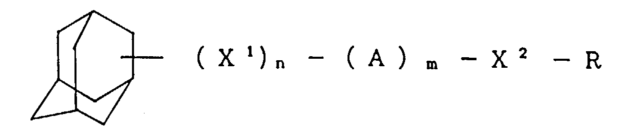 where X¹ and X², which may be identical with or different from each other, represent an ester bond or amide bond, A represents a lower alkylene group in which n = 0 if m = 0 and n = 0 or 1 if m = 1, and R represents a pentapeptide or a physiologically active derivative thereof, provided that when R is enkephalin, X² represents an ester bond. In a further aspect of the present invention, the physiologically active substance is H-Tyr-D.Ala-Gly-Phe-Leu-NH-Ada dihydrate.

As has been described above, adamantyl groups are introduced to the pentapeptide as the physiologically active substance according to the present invention, so that the poor BBB permeability of the physiologically active substance can be improved. Accordingly, it has been shown such a possibility as capable of administrating, by way of hypodermic injection, enkephalins and such that have not hitherto shown analgesic effect unless directly administrated intraventricularly.

As the physiologically active substance used in the present invention, there can be mentioned, for example, pentapeptides from the type of enkephalin, thyrotropine releasing hormone, adrenocorticotropic hormone, cholecystokinin, P-substance and GRF. Specific enkephalin type examples are defined in claims 3 to 9.

In the present invention, one or more of optional positions in the adamantyl group may be substituted with a group for enhancing the BBB permeability of the physiologically active substance. As the specific group, there can be mentioned, for example, lower alkyl group such as methyl group or ethyl group, hydroxyl group, lower alkoxy group, amino group, carboxyl group and carboxy lower alkyl group. It is, however, desirable that the compound is not so much hydrophilic.

According to the present invention, the BBB permeability can be enhanced by introducing the adamantyl group to a substance of poor BBB permeability to provide a possibility that enkephalins and such showing no analgesic effect unless directly administrated intraventricularly.
Fig. 1 is a view illustrating the structural formula of an enkephalin derivative (proto type a-d);
Fig. 2 is a graph illustrating the result of measurement for the in vivo analgesic activity of the derivative a;
Fig. 3 is a graph illustrating the result of measurement for the in vivo analgesic activity of the derivative b;
Fig. 4 is a graph illustrating the result of measurement for in vivo analgesic activity of morphine hydrogen chloride;
Fig. 5 is a graph illustrating the result of measurement for in vivo analgesic activity of the control.

The present inventors have made various studies for improving the BBB permeability of the enkephalin derivative and, as a step therefor, have tried to introduce an adamantyl group for improving the fat solubility.

As a proto type before introducing the adamantyl group, a derivative : H-Tyr-D.Ala-Gly-Phe-Leu-OH in which the second Gly from the N-terminus of enkephalin is substituted with D-Ala was used. For the above-mentioned derivative, it has already been reported that the derivative has a high resistivity to an aminopeptidase and has enhanced analgesic effect.

The following four kinds of derivatives in which the adamantyl group is introduced to the derivative were synthesized, in which Ada represents an adamantane residue.

(A) H-Tyr-D.Ala-Gly-Phe-Leu-O-Ada --- a

At first, Z-Tyr(Bzl)-D-Ala-Gly-NHNH₂ and H-Phe-Leu-O-Ada were synthesized, both of them were condensated by an azide method to obtain a pentapeptide protected at the N-terminus, which was then decapped by catalytic reduction to obtain a derivative a.

(B) H-Tyr-D.Ala-Gly-Phe-Leu-NH-Ada ·2H₂O --- b

The derivative b was obtained by using H-Phe-Leu-NH-Ada instead of H-Phe-Leu-O-Ada and applying the same treatment as in (A) described above.

(C) H-Tyr-D.Ala-Gly-Phe-Leu-O-CH₂-Ada --- c

(D) H-Tyr-D.Ala-Gly-Phe-Leu-O-(CH₂)₂-Ada --- d

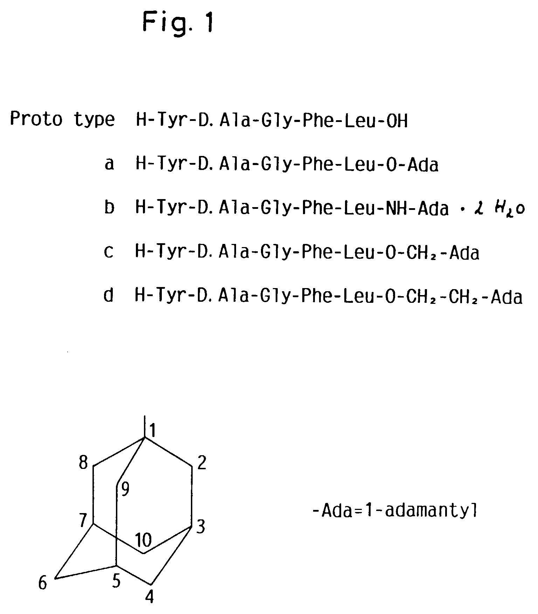

The derivatives c and d are obtained by applying the same treatment as in (A) above by using H-Phe-Leu-O-CH₂-Ada or H-Phe-Leu-O-(CH₂)₂-Ada (refer to Fig. 1).

Descriptions will be made more in details to each of synthesizing steps. In the present specification, abbreviations for amino acid residues and respective protection groups used herein are the followings in accordance with the customary practice in the relevant field.
- Tyr: : L-tyrosine
- Gly: : glycine
- Phe: : L-phenylalanine
- Leu: : L-leucine
- GABA: : γ-amino butyric acid
- D.Ala: : D-alanine
- Z: : benzyloxycarbonyl
- Z(OMe): : p-methoxybenzyloxycarbonyl
- Ada: : 1-adamantyl
- Bzl: : benzyl
- ONp: : p-nitrophenyl ester
- OSu: : N-hydroxy succinic imide ester
- OMe: : methyl ester
- OAda: : 1-adamantyloxy
- NHAda: : 1-adamantyl amino
- NHNH₂: : hydrazide
- DCC: : dicyclohexyl carbodiimide
- DBU: : 1,8-diazabicyclo[5,4,0]-7-undecene
- TFA: : trifluoro acetic acid
- THF: : tetrahydrofuran
- DMA: : N,N-dimethylacetamide
- DMAP: : 4,4-dimethylaminopyridine
- DMF: : dimethylformamide

Further, in the thin layer chromatography (TLC), silica gel is used and the solvent system is as shown below:
Rf₁ = CHCl₃ : MeOH : H₂O (8:3:1)
Rf₂ = CHCl₃ : MeOH (10:1)
Rf₃ = CHCl₃ : MeOH (20:1)
Rf₄ = CHCl₃ : MeOH (50:1)
Rf₅ = CHCl₃

### Example 1

### Preparation of H-Try-D.Ala-Gly-Phe-Leu-OAda

### (1) Preparation of Z-Leu-OAda

Z-Leu-OH (3.00 g, 11.3 mmol) was dissolved in methylene chloride (30 ml), to which adamantan-1-ol (1.9 g, 12.4 mmol), DCC (2.60 g, 12.4 mmol) and DMAP (0.15 g, 1.24 mmol) were added and stirred at 4 °C for 72 hours. After removing the resultant urea derivative by filtration, the solvent was distilled off.The residue was dissolved in ethyl acetate, and the ethyl acetate layer was washed with an aqueous 5% solution of sodium carbonate, an aqueous 10% solution of citric acid, an aqueous saturated solution of sodium chloride and then water successively. After drying with sodium sulfate, ethyl acetate was distilled off. Further, the residue was dissolved in chloroform and then purified with silica gel column chromatography. From the chloroform elution fraction, an oily product showing a single spot was obtained.
Yield: 3.10 g (69%)

### (2) Preparation of Z-Phe-Leu-OAda

Z-Leu-OAda (6.20 g, 15.5 mmol) was dissolved in THF (30 ml), to which two drops of acetic acid and 5% palladium-carbon (2.0 g) were added and a hydrogen gas was passed therethrough for 2 hours. After removing palladium-carbon by filtration, the-filtrate was condensated and the residue was dissolved in DMF (70 ml). Triethylamine (2.14 ml, 15.5 mmol) and Z-Phe-ONp (4.64 g, 15.5 mmol) were added to the solution and stirred for 48 hours. After removing DMF by distillation, the residue was dissolved in ethyl acetate and washed with an aqueous 10% solution of citric acid, an aqueous 5% solution of sodium carbonate, an aqueous saturated solution of sodium chloride and water successively. After drying with sodium sulfate, ethyl acetate was distilled off. Further, the residue was dissolved in chloroform, and an oily product showing a single spot was obtained by silica gel column chromatography using chloroform as an eluting solution.
Yield : 4.25 g (51%)

### (3) Preparation of Z(OMe)-D.Ala-Gly-OMe

Z(OMe)-D.Ala-OH (5.06 g, 20 mmol) was dissolved in DMF and a mixed acid anhydride was prepared from triethylamine (3.04 ml, 22 mmol) and isobutylchloroformate (2.88 ml, 22 mmol). H-Gly-OMe hydrogen chloride (2.07 g, 16.5 mmol) was dissolved in DMF (20 ml) and neutralized with triethylamine (2.30 ml, 16.5 mmol). Both of the solutions were mixed and stirred under ice cooling for 2 hours. After distilling off DMF, the residue was dissolved in ethyl acetate and washed with an aqueous 10% solution of citric acid, an aqueous 5% solution of sodium carbonate, an aqueous saturated solution of sodium chloride and then with water successively. After drying with sodium sulfate, ethyl acetate was distilled off and ether was added to the residue to obtain powder. After collecting the powder by filtration, it was recrystallized from ethyl acetate - ether.
Yield: 3.10 g (58 %), Melting point: 99 - 101°C
Rf₁ : 0.75

| Elemental analysis C₁₅H₂₀N₂O₆ | | | |
|---|---|---|---|
| Calculated value: | C 55.55, | H 6.22, | N 8.64 |
| Measured value : | C 55.41, | H 6.34, | N 8.68 |

### (4) Preparation of Z-Tyr(Bzl)-D.Ala-Gly-OMe

TFA (6.0 ml) - anisole (1.5 ml) were added to Z(OMe)-D.Ala-Gly-OMe (3.00 g, 9.25 mmol) under ice cooling and stirred for one hour. After distilling off TFA, the residue was dissolved in DMF (30 ml) and neutralized with triethylamine (1.28 ml, 9.25 mmol). Z-Tyr(Bzl)-OH (3.81 g, 11.1 mmol) was dissolved in DMF (40 ml) to which a mixed acid anhydride prepared from N-methylmorpholine (1.34 ml, 12.2 mmol) and isobutylchloroformate (1.60 ml, 12.1 mmol) were added and stirred under ice cooling for 2 hours. After distilling off DMF, the residue was dissolved in ethyl acetate, and washed with an aqueous 10% solution of citric acid, an aqueous 5% solution of sodium carbonate, an aqueous saturated solution of sodium chloride and water successively. After drying with sodium sulfate, ethyl acetate was distilled off and isopropyl ether was added to the residue to obtain powder. After collecting it by filtration, it was recrystallized from methanol - ether.
Yield: 3.31 g (65 %), Melting point: 157 - 161°C
Rf₁ : 0.81

| Elemental analysis C₃₀H₃₃N₃O₇ | | | |
|---|---|---|---|
| Calculated value: | C 65.80, | H 6.07, | N 7.67 |
| Measured value : | C 65.33, | H 6.06, | N 7.60 |

### (5) Preparation of Z-Try(Bzl)-D.Ala-Gly-NHNH₂

Z-Tyr(Bzl)-D.Ala-Gly-OMe (3.20 g, 5.8 mmol) was dissolved in DMF (40 ml), to which 80% hydrazine hydrate (3.60 ml, 1.58 mml) was added and stirred for 24 hours. After distilling off DMF, ethanol was added to the residue to obtain powder. After collecting it by filtration, it was recrystallized from DMF - ethanol.
Yield: 3.15 g (98 %), Melting point: 171 - 174°C
Rf₁ : 0.61

| Elemental analysis C₂₉H₃₃N₅O₆.H₂O | | | |
|---|---|---|---|
| Calculated value: | C 61.58, | H 6.24, | N 12.38 |
| Measured value : | C 61.73, | H 6.06, | N 12.86 |

### (6) Preparation of Z-Tyr(Bzl)-D.Ala-Gly-Phe-Leu-OAda

Z-Phe-Leu-OAda (1.00 g, 1.83 mmol) was dissolved in tetrahydrofuran (20 ml), to which 5% palladium-carbon (about 1 g) was added and catalytic reduction was applied by passing a hydrogen gas. 2 hours after, the catalyst was removed by filtration, the solvent was distilled off and the residue was dissolved in DMF (50 ml). An azide ingredient prepared from Z-Tyr(Bzl)-D.Ala-Gly-NHNH₂) (1.30 g, 2.38 mmol) and triethylamine (0.70 ml, 5.0 mmol) were added and stirred at 4°C for 24 hours. After distilling off DMF, water is added to the residue to obtain powder. The power was collected by filtration and then washed with an aqueous 10% solution of citric acid, an aqueous 5% solution of sodium hydrogen carbonate, an aqueous saturated solution of sodium chloride and water successively. After drying, it was recrystallized twice from methanol - ether.
Yield: 1.67 g (85 %), Melting point: 136 - 139°C
Rf₁ : 0.38

| Elemental analysis C₅₄H₆₅N₅O₉.1/2H₂O | | | |
|---|---|---|---|
| Calculated value: | C 69.21, | H 7.17, | N 7.47 |
| Measured value : | C 69.29, | H 7.23, | N 7.33 |

### (7) Preparation of H-Tyr-D.Ala-Gly-Phe-Leu-OAda

Z-Tyr(Bzl)-D.Ala-Gly-Phe-Leu-OAda (250 mg, 0.27 mmol) was dissolved in THF (30 ml), to which 5% palladium-carbon (1 g) was added and catalytic reduction was applied for 5 hours. After removing the catalyst by filtration, the solvent was distilled off and the residue was dissolved in a small amount of chloroform - methanol (10: 1). A single fraction was obtained by silica gel column chromatography using the same solvent as the eluting solution, the solvent was distilled off and ether was added to the residue to obtain powder.
Yield: 85 mg (85 %), Melting point: 110 - 115°C
Rf₁ : 0.53, [α]_{D} = + 159.6° (C=0.2, DMF, 25°C)

| Elemental analysis C₃₉H₅₃N₅O₇.2H₂O | | | |
|---|---|---|---|
| Calculated value: | C 63.31, | H 7.77, | N 9.47 |
| Measured value : | C 63.56, | H 7.66, | N 9.05 |

FAB-MS: 704.0 [M+H]⁺
Amino acid analysis value after hydrolysis with 6N hydrogen chloride
Gly 1.13, Ala 1.03, Leu 1.00, Tyr 0.88, Phe 1.05

### Example 2

### Preparation of H-Tyr-D.Ala-Gly-Phe-Leu-NHAda · 2 H₂O

### (1) Preparation of Z-Leu-NHAda

Z-Leu-OH (1.75 g, 6.6 mmol) was dissolved in DMF (30 ml), to which triethylamine (0.94 ml, 6.8 mmol) and isobutylchloroformate (0.90 ml, 6.8mmol) were added to prepare a mixed acid anhydride. A solution prepared by dissolving 1-aminoadamantane hydrogen chloride (1.88 g, 10 mmol) in DMF (20 ml) and neutralizing with triethylamine (1.38 ml, 10 mmol) was added to the above-mentioned solution and stirred under ice cooling for 2 hours. After distilling off the solvent, the residue was dissolved in ethyl acetate and washed with an aqueous 10% solution of citric aid, an aqueous 5% solution of sodium carbonate, an aqueous unsaturated solution of sodium chloride and water successively. After drying with sodium sulfate, ethyl acetate was distilled off and petroleum ether was added to the residue to obtain powder. After collecting it by filtration, it was recrystallized from ethyl acetate - petroleum ether.
Yield: 2.20 g (84 %), Melting point: 103 - 105°C
Rf₅ : 0.44

| Elemental analysis C₂₄H₃₄N₂O₃ | | | |
|---|---|---|---|
| Calculated value: | C 72.33, | H 8.60, | N 7.03 |
| Measured value : | C 72.02, | H 8.78, | N 6.82 |

### (2) Preparation of Z-Phe-Leu-NHAda

Z-Leu-NHAda (2.20 g, 5.5 mmol) was dissolved in methanol (30 ml), to which two drops of acetic acid and 5% palladium-carbon (about 1 g) were added and catalytic reduction was applied for 2 hours. After removing the catalyst by filtration, the filtrate was concentrated and the residue was dissolved in DMF (20 ml). The solution after the catalytic reduction was added to a DMF solution (20 ml) of a mixed acid anhydride prepared from Z-Phe-OH (1.98 g, 6.63 mmol), triethylamine (1.00 g, 7.29 mmol) and isobutylchloroformate (0.96 ml, 7.29 mmol) and they were stirred under ice cooling for two hours. After distilling off DMF, water was added to the residue to form powder, which was collected by filtration. It was washed with an aqueous 10% solution of citric acid, an aqueous 5% solution of sodium carbonate, an aqueous saturated solution of sodium chloride and water successively. After drying, it was recrystallized from ethyl acetate - petroleum ether.
Yield: 2.20 g (68 %), Melting point: 125 - 127°C
Rf₄ : 0.58

| Elemental analysis C₃₃H₄₃N₃O₄.1/2H₂O | | | |
|---|---|---|---|
| Calculated value: | C 71.45, | H 8.00, | N 7.58 |
| Measured value : | C 71.57, | H 8.08, | N 7.61 |

### (3) Preparation of Z-Tyr(Bzl)-D.Ala-Gly-Phe-Leu-NHAda

Z-Phe-Leu-NHAda (1.00 g, 1.83 mmol) was dissolved in THF (20 ml), to which two drops of acetic acid and 5% palladium-carbon (1 g) were added and catalytic reduction was applied for 4 hours. After removing catalyst by filtration, the solvent was dissolved off and the residue was dissolved in DMF (50 ml). An azide ingredient prepared from Z-Tyr(Bzl)-D.Ala-Gly-NHNH₂ (1.30 g, 2.38 mmol) and triethylamine (0.70 ml, 2.38 mmol) were added and stirred at 4°C for 24 hours. After distilling off DMF, water was added to the residue to obtain powder. The powder was collected by filtration and washed with an aqueous 10% solution of citric acid, an aqueous 5% solution of sodium hydrogen carbonate, an aqueous saturated solution of sodium chloride and water successively. After drying, it was recrystallized from methanol - isopropyl ether.
Yield: 1.39 g (81 %), Melting point: 145 - 149°C
Rf₁ : 0.75

| Elemental analysis C₅₄H₆₆N₆O₈.1/2H₂O | | | |
|---|---|---|---|
| Calculated value: | C 69.28, | H 7.28, | N 8.98 |
| Measured value : | C 69.25, | H 7.14, | N 8.85 |

### (4) Preparation of H-Tyr-D.Ala-Gly-Leu-NHAda

Z-Tyr(Bzl)-D.Ala-Gly-Phe-Leu-NHAda (500 mg, 0.54 mmol) was dissolved in THF (30 ml), to which two drops of acetic acid and 5% palladium-carbon (1 g) were added and catalytic reduction was applied for 4 hours. After removing the catalyst by filtration, the solvent was distilled off and the residue was dissolved in a small amount of chloroform. A fraction showing a single spot was obtained by silica gel column chromatography using chloroform - methanol (10:1) as an eluting solution. After distilling off the solvent, ether was added to the residue to obtain powder.
Yield: 155 mg (41 %), Melting point: 141 - 145°C
Rf₁ : 0.51, [α]_{D} = + 32.5° (C=0.2, DMF, 25°C)

| Elemental analysis C₃₉H₅₄N₆O₉.2H₂O | | | |
|---|---|---|---|
| Calculated value: | C 63.39, | H 7.91, | N 11.37 |
| Measured value : | C 63.76, | H 7.85, | N 11.21 |

FAB-MS: 703.0 [M+H]⁺
Amino acid analysis value after hydrolysis with 6N hydrogen chloride
Gly 1.04, Ala 1.03, Leu 1.00, Tyr 1.00, Phe 1.07

### Example 3

### Preparation of H-Tyr-D.Ala-Gly-Phe-Leu-O-CH₂-Ada and H-Tyr-D.Ala-Gly-Phe-Leu-O-(CH₂)₂-Ada

### (1) Preparation of Z-Leu-O-CH₂-Ada

Z-Leu-OH (2.10 g, 7.9 mmol) was dissolved in methylene chloride (30 ml), to which 1-adamantane methanol (1.31 g, 7.9 mmol), DCC (1.8g, 8.7 mmol) and DMAP (97 mg, 0.79 mmol) were added and stirred at 4°C for 48 hours. After removing the resultant urea derivative by filtration, the solvent was distilled off. The residue was dissolved in ethyl acetate, and ethyl acetate layer was washed with an aqueous 5% solution of sodium carbonate, an aqueous 10% solution of citric acid, an aqueous saturated solution of sodium chloride and water successively. After drying with sodium sulfate, ethyl acetate was distilled off. Further, the residue was dissolved in chloroform and purified by silica gel column chromatography to obtain an oily product showing a single spot.
Yield : 2.43 g (74%)
Rf₃: 0.88
FAB-MS: 414.2 [M+H]⁺

### (1') Preparation of Z-Leu-O-CH₂-CH₂-Ada

The procedures were the same as those in (1) above except for using 1-adamantane ethanol (1.70 g, 9.4 mmol) instead of 1-adamantane methanol.
Yield: 1.85 g (46%)
Rf₅ : 0.63
FAB-MS: 428.0 [M+H]⁺

### (2) Preparation of Z-Phe-Leu-O-CH₂-Ada

Z-Leu-O-CH₂-Ada (2.18 g, 5.27 mmol) was dissolved in THF (20 ml), to which 5% palladium-carbon (about 2 g), acetic acid (2 ml) were added and catalytic reduction was applied for 10 hours. After removing the palladium-carbon by filtration, the filtrate was concentrated and the residue was dissolved in DMF (20 ml). Triethylamine (0.74 ml, 5.27 mmol) and Z-Phe-ONP (2.21 g, 5.27 mmol) and N-hydroxybenzotriazole (0.36 g, 2.64 mmol) were added and stirred for 24 hours. After distilling off DMF, the residue was dissolved in ethyl acetate and washed with an aqueous 5% solution of sodium carbonate, an aqueous 10% solution of citric acid, an aqueous saturated solution of sodium chloride and water successively. After drying with sodium sulfate, ethyl acetate was distilled off. Further, the residue was dissolved in chloroform and purified by silica gel column chromatograph using chloroform - methanol (20:1) as an eluting solution to obtain an oily product showing a single spot.
Yield: 2.19 g (99%)
Rf₄ : 0.46
FAB-MS: 561.3 [M+H]⁺

### (2') Preparation of Z-Phe-Leu-O-(CH₂)₂-Ada

The procedures were the same as those in (2) above except for using Z-Leu-O-CH₂-CH₂-Ada (1.85 g, 4.33 mmol) instead of Z-Leu-O-CH₂-Ada.
Yield: 2.49 g (100%)
Rf₅ : 0.15
FAB-MS: 575.0 [M+H]⁺

### (3) Preparation of Z-Tyr(Bzl)-D-Ala-Gly-Phe-Leu-O-CH₂-Ada

Z-Phe-Leu-O-CH₂-Ada (2.0 g, 3.57 mmol) was dissolved in tetrahydrofuran (15 ml), to which 5% palladium-carbon (about 2 g) and acetic acid (1 ml) were added and catalytic reduction was applied for 5 hours. After removing the catalyst by filtration, the filtrate was concentrated. The residue was dissolved in DMF (10 ml), to which an azide ingredient prepared from Z-Tyr(Bzl)-D.Ala-Gly-NHNH₂ (1.60 g, 2.92 mmol) and triethylamine (0.27 ml, 1.95 mmol) were added and stirred at 4°C for 48 hours. After distilling off DMF, the residue was dissolved in ethyl acetate and washed with aqueous 5% solution of sodium carbonate, an aqueous 10% solution of citric acid, an aqueous saturated solution of sodium chloride and water successively. After drying with sodium sulfate, ethyl acetate was distilled off under a reduced pressure and ether was added to the residue to obtain powder. Further, it was dissolved in chloroform and purified by silica gel column chromatography using chloroform - methanol (20:1) as an eluting solution, to obtain a product showing a single spot, which was recrystallized from methanol ether.
Yield: 0.70 g (38 %), Melting point: 158 - 162°C
Rf₃: 0.34 [α]_{D} = -17.2° (C=0.5, DMF)

| Elemental analysis: C₅₅H₆₇N₅O₉.1/2H₂O | | | |
|---|---|---|---|
| Calculated value: | C 69.45, | H 7.21, | N 7.36 |
| Measured value : | C 69.61, | H 7.29, | N 7.79 |

### (3') Preparation of Z-Tyr(Bzl)-D.Ala-Gly-Phe-Leu-O-(CH₂)₂-Ada

Z-Phe-Leu-O-(CH₂)₂-Ada (1.15 g, 2 mmol) was dissolved in tetrahydrofuran (15 ml), to which 5% palladium-carbon (about 2 g) and 5 ml of acetic acid were added and catalytic reduction was applied for 5 hours. After removing the catalyst by filtration, the filtrate was concentrated. The residue was dissolved in DMF (10 ml), to which an azide ingredient prepared from Z-Tyr(Bzl)-D.Ala-Gly-NHNH₂ (1.2 g, 2.19 mmol) and triethylamine (0.28 ml, 2 mmol) were added and stirred at 4°C for 48 hours. After distilling off DMF, the residue was dissolved in ethyl acetate and washed with aqueous 5% solution of sodium carbonate, an aqueous 10% solution of citric acid, an aqueous saturated solution of sodium chloride and water successively. After drying with sodium sulfate, ethyl acetate was distilled off under a reduced pressure and ether was added to the residue, to obtain powder. Further, it was dissolved in chloroform and purified by silica gel column chromatography using chloroform - methanol (30:1) as an eluting solution, to obtain a product showing a single spot, which was recrystallized from methanol ether.
Yield: 0.84 g (51 %), Melting point: 148 - 151°C
Rf₃: 0.31, [α]_{D} = -13.3° (C=0.6, DMF)

| Elemental analysis: C₅₆H₆₉N₅O₉.1.5H₂O | | | |
|---|---|---|---|
| Calculated value: | C 68.41, | H 7.38, | N 7.12 |
| Measured value : | C 68.44, | H 7.09, | N 7.46 |

### (4) Preparation of H-Try-D.Ala-Gly-Phe-Leu-O-CH₂-Ada

Z-Tyr(Bzl)-D.Ala-Gly-Phe-Leu-O-CH₂-Ada (283 mg, 0.3 mmol) was dissolved in acetic acid (10 ml), to which 5% palladium-carbon (1 g) were added and catalytic reduction was conducted for 5 hours. After removing the catalyst by filtration, the filtrate was diluted with water and then freeze-dried. The freeze drying product was dissolved in 5% acetic acid, placed in a Diaion HP-20 column and washed with 5% acetic acid (200 ml). Then, it was eluted with gradient elution using 5% acetic acid (200 ml) and acetonitrile (200 ml) and the main fraction was concentrated and freeze-dried, to obtain white feather like crystals.
Yield: 135 mg (56 %)
Rf₁: 0.30 [α]_{D} = +6.3° (C=0.2, DMF)
FAB-MS: 718.0 [M+H]⁺

| Elemental analysis: C₄₁H₅₄N₅O₇·1.5H₂O | | | |
|---|---|---|---|
| Calculated value: | C 65.14, | H 7.60, | N 9.27 |
| Measured value : | C 65.15, | H 7.61, | N 9.39 |

### (4') Preparation of H-Tyr-D.Ala-Gly-Phe-Leu-O-(CH₂)₂-Ada

Z-Tyr-(Bzl)-D.Ala-Gly-Phe-Leu-O-(CH₂)₂-Ada (287 mg, 0.3 mmol) was dissolved in 10 ml of acetic acid, to which 1 g of 5% palladium-carbon was added and catalytic reduction was applied for 5 hours. After removing the catalyst by filtration, the filtrate was diluted with water and then freeze-dried. The freeze drying product was dissolved in 5% acetic acid, placed on a Diaion HP-20 column and washed with 5% acetic acid (200 ml). Then, it was eluted by gradient elution using 5% acetic acid (200 ml) and acetonitrile (200 ml). The main fraction was concentrated and freeze-dried to obtain white feather-like crystals.
Yield: 110 mg (50 %), Melting point : 117 - 122°C
Rf₁: 0.28 [α]_{D} = +34.9° (C=0.2, DMF)
FAB-MS: 732.0 [M+H]⁺

| Elemental analysis: C₄₁H₅₇N₅O₇·1.5H₂O | | | |
|---|---|---|---|
| Calculated value: | C 64.88, | H 7.97, | N 9.23 |
| Measured value : | C 64.61, | H 7.72, | N 8.91 |

### Example 4

Adamantylated enkephalin derivatives a and b obtained in Examples 1 and 2 were examined for in vivo analgesic effect and in vitro suppressing effect to shrinkage of extirpated intestine of guinea pig by electric stimulations.

### Measurement for in vivo analgesic activity

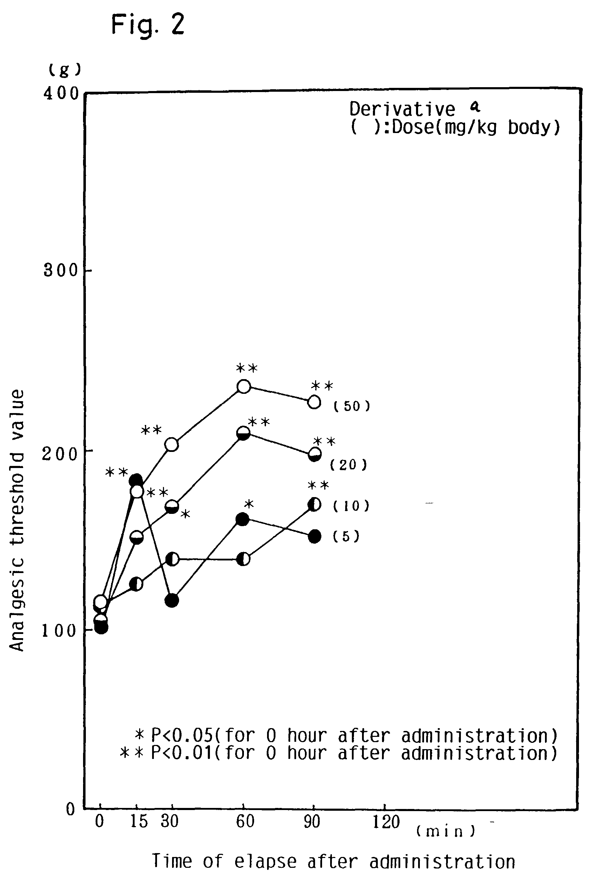

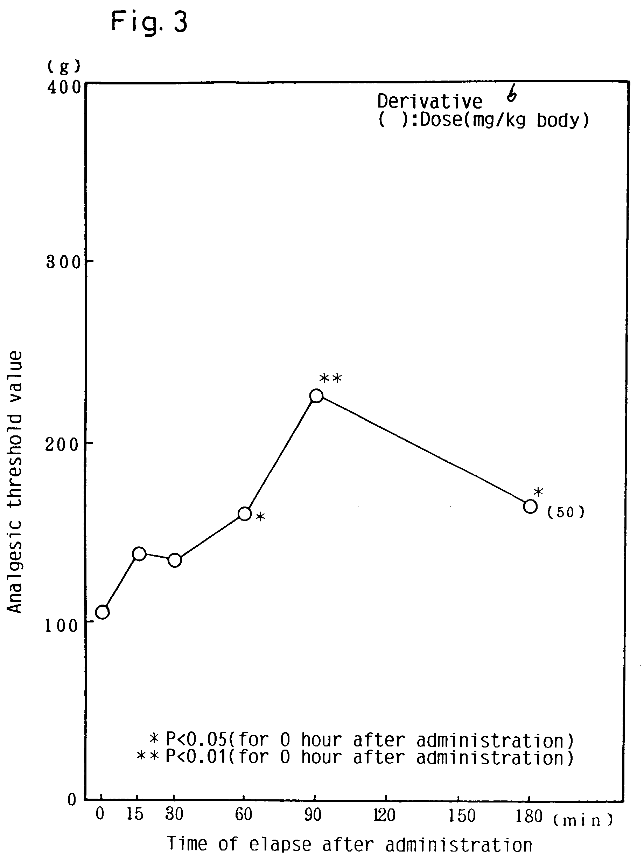

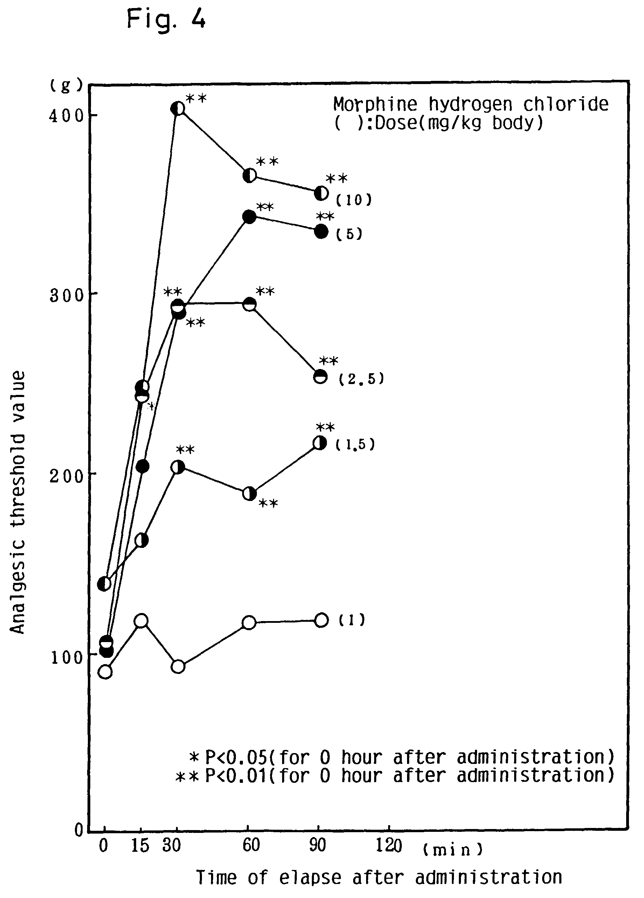

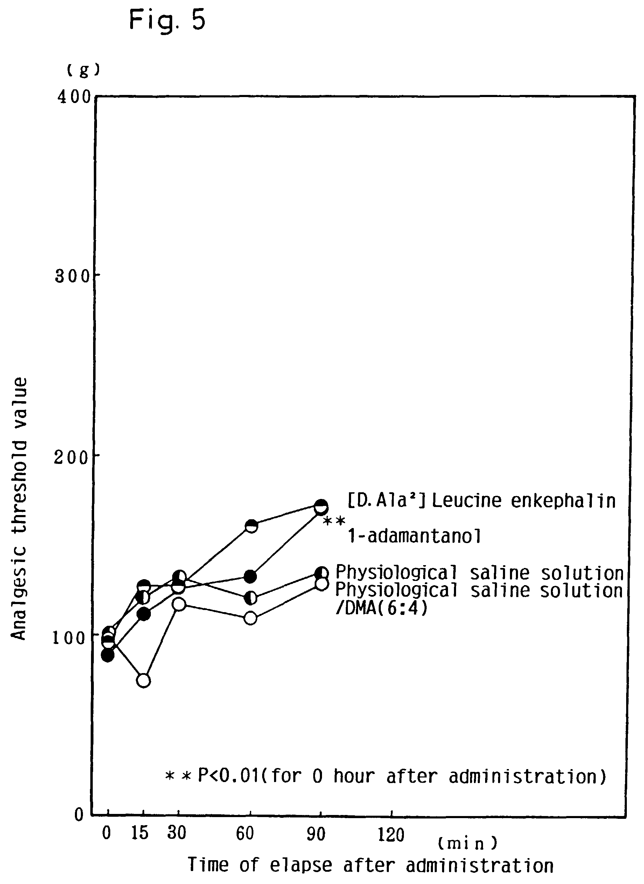

Adamantylated enkephalin derivatives a and b were subcutaneously administrated to a mouse, and an analgesic effect in the central nervous system was measured by Randall Selitto method. That is, the derivatives a and b were dissolved in a mixed solution of DMA : physiological saline solution (4:6) and about 100 µl of the solution was subcutaneously administrated to a mouse (dd-Y series: about 20 g) (1 - 50 mg/kg), and the analgesic effect was measured on every predetermined time interval after the administration by the Randall Selitto method. Morphine hydrogen chloride was used as a comparative drug and [D-Ala²] leucine enkephalin, 1-adamantanol, physiological saline solution and mixed solvent of physiological saline solution + DMA (6:4) were used as controls, respectively. The test results are shown in Fig. 2 (derivative a), Fig. 3 (derivative b), Fig. 4 (morphine hydrogen chloride) and Fig. 5 (control).

The derivatives a and b having adamantyl group introduced on the C-terminus showed analgesic activity inferior to morphine hydrogen chloride but superior to the controls.

### Measurement for in vitro analgesic activity

A suppressing effect for the shrinkage of extirpated intestine of guinea pig of the adamantylated enkephalin derivatives a and b was measured in accordance with the method of Kosterlitz (H.W. Kosterlitz, A.A. Watterfield, Ann. Rev. Pharmacol., 15, 29, (1975)). Further, morphine hydrogen chloride was used as a comparative drug and [D-Ala²] leucine enkephalin was used as a control. The test results are shown in Table 1.

**Table 1**

| Compound | ED₅₀ (mol) (mean ± s.e.m.) | Relative Potency |
|---|---|---|
| Morphine hydrogen chloride | 3.2 ± 0.43 × 10⁻⁸ (n=17) | 1.00 |
| [D-Ala²] Leucine enkephalin | 3.6 ± 1.17 × 10⁻⁸ (n=7) | 0.89 |
| Derivative a | 9.4 ± 4.82 × 10⁻⁸ (n=7) | 0.34 |
| Derivative b | 1.5 ± 0.71 × 10⁻⁸ (n=5) | 2.13 |

Each of the derivatives a and b showed suppression for shrinkage each of which was antagonized by naloxon. That is, it can be seen that the derivatives have morphine-like activity irrespective of introduction of the adamantyl group.

From the test results described above, it can be confirmed that the adamantylated enkephalins show morphine-like analgesic effect by hypodermic administration. This shows improvement in the BBB permeability and clinical application as a hypodermic injection solution is expected if the safety is confirmed.

From the results described above, it is shown that the BBB permeability is improved by the introduction of the adamantyl group while keeping the physiological activity and similar effects can also be expected to analogous derivatives. However, since it may be considered that the activity is lost depending on the position of introducing the adamantyl group in view of the nature of the physiological active substance, a sufficient care is necessary upon introduction.

## Claims

1. A physiologically active substance represented by the general formula: 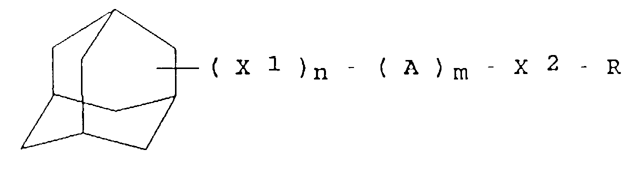 where X¹ and X², which may be identical with or different from each other, represent an ester bond, or amide bond, A represents a lower alkylene group in which n = 0 if m = 0, n = 0 or 1 if m = 1, and R represents a pentapeptide, or a physiologically active derivative thereof, provided that when R is enkephalin, X² represents an ester bond.

2. The substance of Claim 1 wherein X² represents an ester bond and R represents enkephalin.

3. The substance of Claim 1 which is H-Tyr-D.Ala-Gly-Phe-Leu-O-Ada.

4. The substance of Claim 1 which is H-Tyr-D.Ala-Gly-Phe-Leu-O-Ada dihydrate.

5. The substance of Claim 1 which is H-Tyr-D.Ala-Gly-Phe-Leu-O-CH₂-Ada.

6. The substance of Claim 1 which is H-Tyr-D.Ala-Gly-Phe-Leu-O-CH₂-Ada·1.5H₂O.

7. The substance of Claim 1 which is H-Tyr-D.Ala-Gly-Phe-Leu-O-(CH₂)₂-Ada.

8. The substance of Claim 1 which is H-Tyr-D.Ala-Gly-Phe-Leu-O-(CH₂)₂-Ada·1.5H₂O.

9. A physiologically active substance which is H-Tyr-D.Ala-Gly-Phe-Leu-NH-Ada dihydrate.

## Patentansprüche

1. Physiologisch aktive Substanz gemäß der allgemeinen Formel: 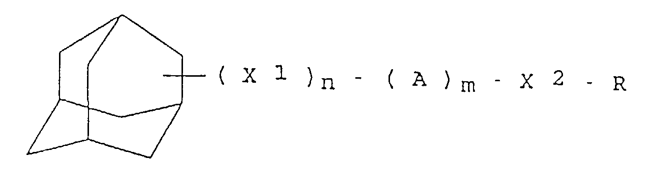 wobei X¹ und X², die jeweils miteinander identisch oder voneinander verschieden sein können, eine Ester- oder Amid-Bindung repräsentieren, A eine Niederalkylen-Gruppe repräsentiert, wobei n = 0, wenn m = 0 ist, n= 0 oder 1, wenn m = 1 ist, und R ein Pentapeptid oder ein physiologisch aktives Derivat davon repräsentiert, vorausgesetzt, daß für den Fall, daß es sich bei R um ein Enkephalin handelt, X² eine Esterbindung repräsentiert.

2. Substanz nach Anspruch 1, wobei X² eine Esterbindung und R ein Enkephalin repräsentiert.

3. Substanz nach Anspruch 1, welche H-Tyr-D.Ala-Gly-Phe-Leu-O-Ada ist.

4. Substanz nach Anspruch 1, welche H-Tyr-D.Ala-Gly-Phe-Leu-O-Ada-Dihydrat ist.

5. Substanz nach Anspruch 1, welche H-Tyr-D.Ala-Gly-Phe-Leu-O-CH₂-Ada ist.

6. Substanz nach Anspruch 1, welche H-Tyr-D.Ala-Gly-Phe-Leu-O-CH₂-Ada·1,5H₂O ist.

7. Substanz nach Anspruch 1, welche H-Tyr-D.Ala-Gly-Phe-Leu-O-(CH₂)₂-Ada ist.

8. Substanz nach Anspruch 1, welche H-Tyr-D.Ala-Gly-Phe-Leu-O-(CH₂)₂-Ada·1,5H₂O ist.

9. Physiologisch aktive Substanz, welche H-Tyr-D.Ala-Gly-Phe-Leu-NH-Ada-Dihydrat ist.

## Revendications

1. Substance physiologiquement active représentée par la formule générale : 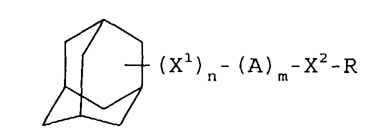 dans laquelle X¹ et X², qui peuvent être identiques ou différents, représentent chacun une liaison ester ou une liaison amide, A représente un groupe alkylène inférieur, n vaut 0 si m vaut 0, n vaut 0 ou 1 si m vaut 1, et R représente un pentapeptide, ou un de ses dérivés physiologiquement actifs, du moment que lorsque R est l'enképhaline, alors X² représente une liaison ester.

2. Substance selon la revendication 1, dans laquelle X² représente une liaison ester et R représente l'enképhaline.

3. Substance selon la revendication 1, qui est le H-Tyr-D.Ala-Gly-Phe-Leu-O-Ada.

4. Substance selon la revendication 1, qui est le H-Tyr-D.Ala-Gly-Phe-Leu-O-Ada dihydraté.

5. Substance selon la revendication 1, qui est le H-Tyr-D.Ala-Gly-Phe-Leu-O-CH₂-Ada.

6. Substance selon la revendication 1, qui est le H-Tyr-D.Ala-Gly-Phe-Leu-O-CH₂-Ada.1,5H₂O.

7. Substance selon la revendication 1, qui est le H-Tyr-D.Ala-Gly-Phe-Leu-O-(CH₂)₂-Ada.

8. Substance selon la revendication 1, qui est le H-Tyr-D.Ala-Gly-Phe-Leu-O-(CH₂)₂-Ada.1,5H₂O.

9. Substance physiologiquement active, qui est le H-Tyr-D.Ala-Gly-Phe-Leu-NH-Ada dihydraté.
